# EUROPÄISCHE PATENTSCHRIFT

(11) **EP 0 943 676 B1**
(45) Veröffentlichungstag und Bekanntmachung des Hinweises auf die Patenterteilung: **21.12.2005**
(21) Anmeldenummer: 99100972.1
(22) Anmeldetag: 21.01.1999
(51) Int. Cl.: C12M 1/00

(54) **Verfahren zur Verschlusskontrolle für Brut- und Trockenschränke sowie Brut- oder Trockenschrank**
Incubator and a method of controlling the closing of an incubator
Incubateur et procédé de commande de la fermeture d'un incubateur

(30) Priorität: 30.01.1998 DE 19803601
(43) Veröffentlichungstag der Anmeldung: 22.09.1999
(73) Patentinhaber: Kendro Laboratory Products GmbH, 63505 Langenselbold (DE)
(72) Erfinder: Jelinski, Sonja, 63594 Hasselroth (DE); Ferger, Stefan, 63691 Ranstadt (DE); Langen, Harald, 64832 Babenhausen (DE)
(74) Vertreter: Lang, Friedrich

(56) Entgegenhaltungen:
- US-A- 4 336 329
- US-A- 4 706 478
- US-A- 4 771 269
- US-A- 5 519 188

## Beschreibung

Die Erfindung betrifft ein Verfahren zum kontrollierten Verschließen wenigstens einer Innentür und einer Außentür eines Brut- oder Trockenschrankes sowie einen Brut- oder Trockenschrank.

Ein solcher Brutschrank ist beispielsweise aus der DE 29 24 446 C2 bzw. der US 4,336,329 bekannt.

Aus dem Prospekt 3C 2.89/VN Ko der W.C. Heraeus GmbH, Seite 6 mittlere Spalte ist es bekannt, in Begasungsbrutschränken Innen-Türen bzw. transparente Türen einzusetzen, um das Innere von Nutzraumsegmenten beim Öffnen der Außentür vor Wärme-, Feuchte- und GasVerlusten zu bewahren; anhand dieses Prospektes ist erkennbar, daß zwischen Nutzraum und Außentür eine zusätzliche transparente Tür vorgesehen ist.

Als problematisch erweist sich die Kontrolle des Öffnungszustandes solcher Glastüren beim Verschließen der Außentür, da für ihren Schließzustand eine erhöhte Aufmerksamkeit bzw. eine zusätzliche elektrische Kontrollanzeige erforderlich ist, die einen erhöhten technischen Aufwand durch zusätzliche Bauteile mit sich bringt.

Weiterhin ist aus der US 55 19 188 ein Brutschrank mit einer äußeren Tür und einem durch eine innere Tür abschließbaren Behandlungsraum bekannt, wobei eine Kontrolleinheit zur Steuerung von Heizelementen vorgesehen ist; der Eingang der Kontrolleinheit ist mit einem Türschalter zur Kontrolle der äußeren Tür und einem Temperatur-Sensor für den Behandlungsraum verbunden, wobei Feuchtigkeit und Temperaturverteilung im Behandlungsraum von der Kontrolleinheit gesteuert werden. Auch bei dieser Anordnung ist ein erhöhter technischer Aufwand erforderlich.

Aufgabe der Erfindung ist es, eine Möglichkeit zur sicheren Verschluß-Kontrolle der Innentür von Trocken- bzw. Brutschränken zu schaffen, wobei eine möglichst einfache Ausgestaltung ohne erhöhten technischen Aufwand angestrebt werden soll.

Die Aufgabe wird verfahrensgemäß dadurch gelöst, daß die mittels seitlichem Scharnier im Öffnungsbereich des Schrankes um ihre Achse schwenkbar gehaltene Innentür auf ihrer der Schwenkachse gegenüberliegenden Seite mittels einer mit klappbarem Handgriff versehenen, drehbar gelagerten starren Verschluß-Zunge eines Verriegelungselements durch Drehung der Zunge in einen Arretierungsspalt eines Verschluß-Elements soweit verriegelt wird, daß der zuvor als Distanzelement zwischen Verschluß-Element und Außentür wirkende Handgriff den Auflagebereich des Verschlußelements verläßt und eine zuvor bestehende Blockade der Schließung der Außentür aufgehoben wird.

Ein wesentlicher Vorteil ist in der einfachen Handhabung sowie vereinfachten Fertigung und Wartung zu sehen, da hier keine komplizierten Schließmechanismen und Signalausgaben erforderlich sind.

In einer bevorzugten Ausführungsform des Verfahrens wird beim Einschwenken der Zunge in das Verschlußelement diese zusammen mit der Innentür durch einen sich in Verriegelungsrichtung wenigstens teilweise verengenden Schlitz als Arretierungsspalt im Verschlußelement in Richtung Innenraum gepreßt.

Hierzu erweist es sich als vorteilhaft, daß die Innentür gegen ein die Öffnung im Nutzraum umgebendes, umlaufendes Dichtelement gepreßt wird, so daß der Nutzraum atmosphärisch abgeschlossen ist, jedoch durch die - vorzugsweise als Glastür aufgebaute - Innentür von außen beobachtet werden kann.

Die Aufgabe wird vorrichtungsgemäß für einen Brut- oder Trocken-Schrank mit wenigstens einer zwischen Nutzraum und Außentür angeordneten Innentür, bei dem beide Türen jeweils durch ein seitliches Scharnier um eine vertikale Schwenk-Achse drehbar gehalten sind, dadurch gelöst, daß zur Verriegelung der Innentür im seitlichen Frontbereich des Schrankes gegenüberliegend der Schwenk-Achse ein Verschlußelement mit einem sich wenigstens teilweise verengenden Schlitz als Arretierungsspalt vorgesehen ist, in den eine mit umklappbarem Handgriff versehene, im freien Schwenkbereich der Innentür drehbar gelagerte starre Verriegelungs-Zunge eines Verriegelungselements eingreifbar angeordnet ist, wobei der Handgriff in der Öffnungsposition der Innentür zwischen Verschlußelement und Außentür als Distanzelement wirkt und durch seine Auflage auf dem Verschlußelement ein Schließen der Außentür blockiert, wobei die Verriegelungs-Zunge bei Eingriff in den Schlitz als Arretierungsspalt mittels Drehbewegung bis zum Anschlag innerhalb des Schlitzes so führbar ist, daß sich der Handgriff außerhalb des Auflagebereichs des Verschlußelements befindet und eine Blockade der Außentür bei ihrem Schließvorgang verhindert.

Neben verringertem Herstellungs- und Wartungsaufwand erweist es sich weiterhin als vorteilhaft, daß nach der erfindungsgemäßen Vorrichtung auch weniger geübtes Bedienungspersonal auf einfache und anschauliche Weise für die Dichtigkeit des Trocken- bzw. Begasungsbrutschranks sorgen kann. Aufgrund der außerhalb des Nutzraums befindlichen Verriegelungsmechanik ist eine einfache und sichere Desinfektion möglich.

In einer bevorzugten Ausgestaltung des Brut- oder Trockenschrankes ist der drehbar angeordnete Handgriff als umklappbares, federbelastetes Distanzelement ausgebildet, das diametral gegenüber der Verriegelungs-Zunge angeordnet ist; dabei schließt die Grifffläche mit der Ebene der Innentür näherungsweise einen Winkel von 70° ein, der sowohl bei geöffneter als auch bei geschlossener Innentür durch Anpressen der Außentür gegen den Handgriff nahezu auf einen Winkel im Bereich von 0 bis 5° verkleinert wird; dabei erweist es sich als besonders vorteilhaft, daß von der Außentür keinerlei Kraft auf die drehbare Lagerung von Zunge und Handgriff ausgeübt werden kann.

Der Handgriff wirkt im entriegelten Zustand der Verriegelungs-Zunge wenigstens zum Teil als ein zwischen dem Verschlußelement und der Außentür befindliches Distanzelement; im verriegelten Zustand der Verriegelungs-Zunge befindet sich der Handgriff näherungsweise in einer Ebene mit der Außenkontur des Verschlußelements.

Als vorteilhaft erweist sich hierbei die Doppelfunktion des Handgriffs mit Verriegelungs-Zunge als Schließ- und Kontrollelement.

Eine erneute Öffnung des Nutzraumes ist durch Drehung des Handgriffs im Uhrzeigersinn möglich.

Aufgrund der rein mechanischen Sicherungsfunktion handelt es sich hierbei um eine besonders preiswerte sowie übersichtliche und zuverlässig zu handhabende Verschlußvorrichtung für Innentüren von Trocken- bzw. Begasungsbrutschränken.

In einer bevorzugten Ausführungsform besteht die Innentür aus transparentem Werkstoff, vorzugsweise Glas, so daß der Nutzraum vorteilhafterweise ohne atmosphärische Beeinflussung von außen optisch überwacht werden kann.

Es ist in einer weiteren vorteilhaften Ausgestaltung möglich, den Nutzraum durch wenigstens eine horizontale Zwischenwand mit einem Dichtelement an der Frontseite in zwei oder mehr Abschnitte zu unterteilen, die jeweils mit einer Innentür abschließbar sind; dabei weist jede Innentür ein Verriegelungselement auf, das beim Schließen mit seiner Verriegelungs-Zunge in ein jeweils entsprechendes Verschlußelement im seitlichen Randbereich des Schrank-Gehäuseteils eingreift.

Als besonders vorteilhaft erweist es sich bei einer solchen Anordnung, daß bestimmte Abschnitte des Nutzraums partiell beschickt werden können, während die übrigen Abschnitte des Nutzraumes vor Wärme, Feuchte- und Gasverlusten bewahrt werden.

Im folgenden ist der Gegenstand der Erfindung anhand der Figuren 1, 2, 3a, 3b, 4a und 4b näher erläutert.
Figur 1 zeigt in einer schematischen Darstellung die perspektivische Ansicht eines Trocken- bzw. Begasungsbrutschrankes mit geöffneter Innentür;
Figur 2 zeigt schematisch eine vereinfachte Frontalansicht des geöffneten Trocken- bzw. Begasungsbrutschrankes mit dem drehbaren Handgriff und Verschlußelement, wobei auf die Darstellung des Bedienungsteils verzichtet wird;
Die Figuren 3a und 3b zeigen die Wirkung des Handgriffs bei unverschlossener Innentür, wobei zwischen Innen- und Außentür der Handgriff auf dem Verschlußelement zu liegen kommt und somit als Distanzelement gegenüber der Außentür wirkt; Innentür und Außentür sind nur bruchstückhaft dargestellt; der zur Befestigung des Verschlußelements vorgesehene Randbereich des Gehäuseteils ist in Figur 3b symbolisch durch eine gestrichelte Linie als Teil der Oberfläche angedeutet.
Die Figuren 4a und 4b zeigen ähnlich wie die Figuren 3a, 3b bruchstückhaft einen Teil der Innentür mit aufgebrachtem Handgriff und Verschlußelement in der arretierten Position, wobei jedoch Handgriff und Verschlußelement in einer Ebene liegen und somit die Wirkung des Handgriffs als Distanzelement nicht mehr existiert.

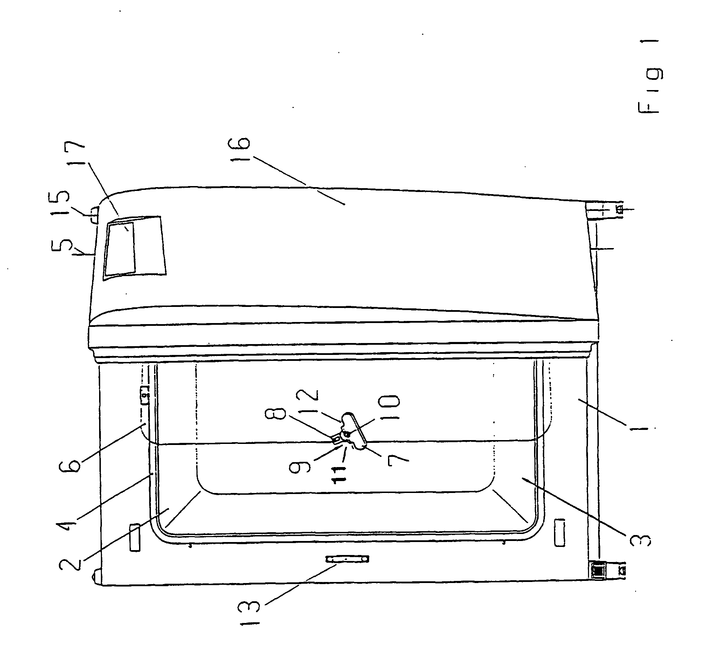

Anhand Figur 1 ist Gehäuseteil 1 mit dem darin befindlichen Nutzraum 2 erkennbar, wobei die frontale Öffnung 3 des Nutzraumes in ihrem Randbereich von einem zur Frontseite gerichteten umlaufenden Dichtelement 4 umgeben ist; die Öffnung 3 ist durch eine mittels Scharniere im seitlichen Randbereich gehaltene Innentür 6 verschließbar, wobei diese um eine vertikale Achse 5 schwenkbar angeordnet ist. An dem der Achse 5 gegenüberliegenden Seitenteil der Innentür 6 befindet sich ein aus Handgriff 7 und Verriegelungs-Zunge 8 bestehendes Verriegelungselement 9, dessen Zunge 8 beim Verschließen der Innentür 6 in einen als Schlitz ausgebildeten Arretierungsspalt eines Verschlußelements 13 durch Drehung einführbar ist; in dieser Figur ist Verriegelungselement 9 in seiner Öffnungs-Position dargestellt.

Das Verriegelungselement 9 ist in einer hier nicht sichtbaren Öffnung des freien Seitenrandes der Innentür 6 um Drehachse 10 drehbar gelagert, wobei Handgriff 7 an einer Nabe 11 umklappbar gehalten ist; dabei ist sichergestellt, daß durch Drehbewegung von Handgriff 7 über einen formschlüssigen Eingriff in Nabe 11 die Drehbewegung auf Verriegelungs-Zunge 8 übertragen wird; somit kann nach Anpressen der Innentür 6 an Dichtelement 4 der frontalen Öffnung 3 von Gehäuseteil 1 durch Drehung von Handgriff 7 entgegen dem Uhrzeiger-Sinn die Verriegelungs-Zunge 8 in den Arretierungsspalt zwischen dem Verschlußelement 13 (Innenseite) und der Oberfläche des Randbereichs eingeführt werden. Handgriff 7 ist im Verhältnis zur Ebene der Innentür 6 im Winkelbereich von ca. 3 bis 70° umklappbar, wobei durch eine hier nicht sichtbare Schraubenfeder entlang der Umklappachse 12 eine Aufstell- bzw. Aufrechtbewegung des Handgriffs gegenüber der Ebene der Innentür 6 vorgenommen wird; eine plötzliche Belastung durch Anpressen beim versuchten Schließen der Außentür führt aufgrund der Abwinkelung des Handgriffs stets zu einer Schließung des Winkels von ca. 70° auf ca. 3°, so daß bei einem versuchten Schließen der um eine vertikale Achse 15 schwenkbar angeordneten Außentür 16 Handgriff 7 in eine Position von ca. 3° umgeklappt wird; dabei kommt Handgriff 7 auf der Außenkontur von Verschlußelement 13 zu liegen, so daß Handgriff 7 als Distanzelement zwischen Verschlußelement 13 und der Innenkontur von Außentür 16 wirkt. Auf diese Weise tritt stets eine Belastung von Verschlußelement 13 bzw. dem umklappbaren Handgriff 7 auf, während das Lager entlang der Drehachse 10 der Innentür 6 unbelastet bleibt. Es ist somit sichergestellt, daß ein versehentliches Schließen der Außentür keinerlei Beschädigungen an der Innentür hervorruft, sofern diese nicht verriegelt ist.

Weiterhin ist anhand Figur 1 das Bedienungsteil 17 mit Anzeige und Einstellelementen erkennbar.

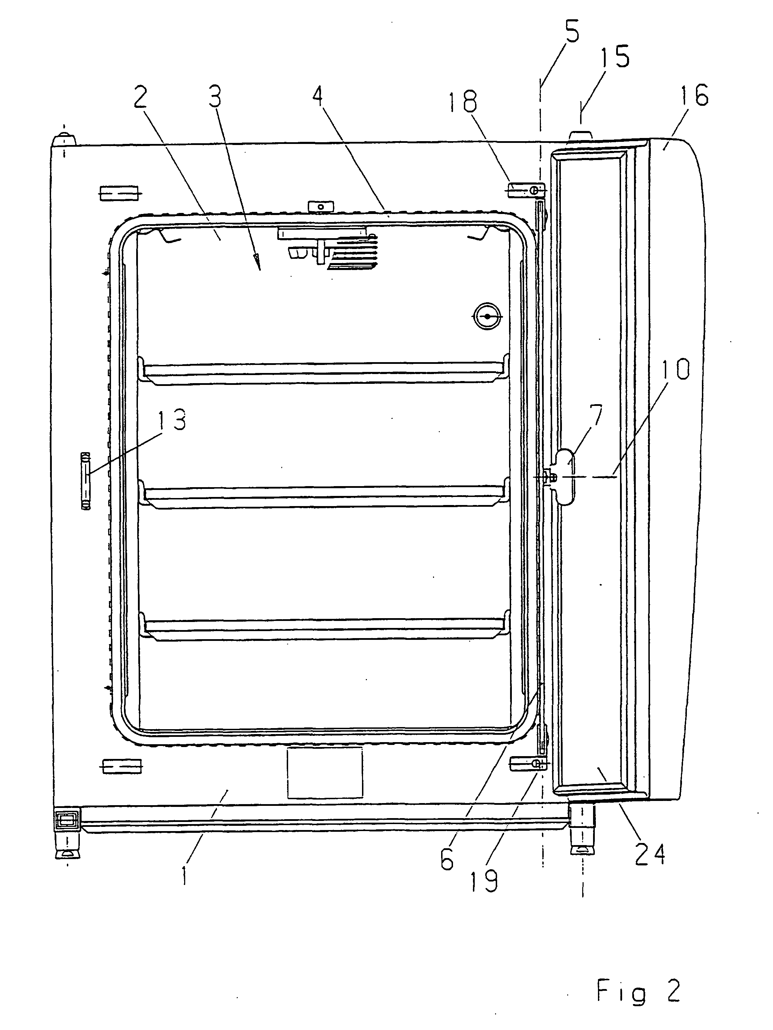

Anhand Figur 2 ist Gehäuseteil 1 des Begasungsbrut- bzw. Trockenschranks erkennbar, wobei Öffnung 3 durch Innentür 6 - vorzugsweise aus transparentem Werkstoff, wie z.B. Glas, und Außentür 16 verschließbar ist. Innentür 6 und Außentür 16 sind hier im geöffneten Zustand dargestellt, wobei Innentür 6 um Achse 5 schwenkbar in den Scharnieren 18 und 19 gelagert ist, während Außentür 16 um Schwenkachse 15 mit ihren Scharnieren drehbar gelagert ist. Die Innenwald der Außentür 16 ist mit Ziffer 24 bezeichnet. Innentür 6 ist an ihrem der Schwenk-Achse 5 gegenüberliegenden Seitenrand mit einem Verriegelungselement 9 versehen, das mit Hilfe der starren drehbaren Verriegelungs-Zunge 8 in Verschlußelement 13 auf dem Seiten-Rand des Gehäuseteils 1 arretierbar angeordnet ist.

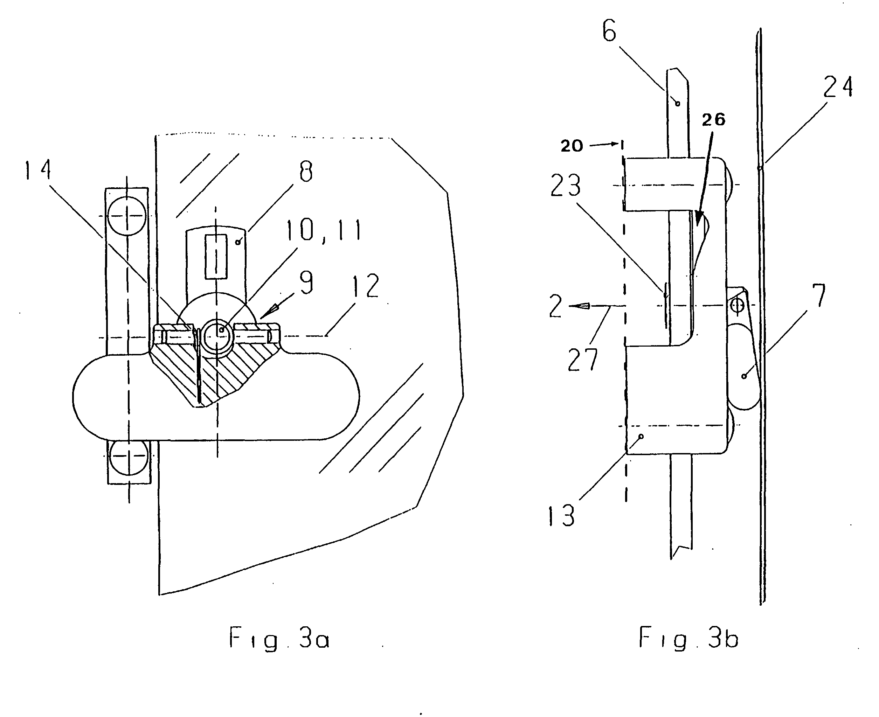

Gemäß Figur 3a zeigt während des geöffneten Zustandes der Innentür 6 eine starre Verriegelungs-Zunge 8 des entlang Achse 10 drehbar angeordneten Verriegelungselements 9 nach oben, während der zugehörige Handgriff 7 um eine senkrecht zur Drehachse 10 angeordnete Achse 12 umklappbar ist, wobei jedoch durch Formschluß zwischen dem Handgriff und der starr mit Verriegelungs-Zunge 8 verbundenen Nabe 11 die Drehbewegung der Verriegelungs-Zunge 8 mittels Handgriff jederzeit gewährleistet ist.

Wie Figur 3a zeigt, kommt der Handgriff 7 auf der nach außen gerichteten Oberfläche von Verschlußelement 13 zu liegen, so daß gemäß Figur 3b der Handgriff 7 als Distanzelement zwischen Verschlußelement 13 und der Innenwand 24 der Außentür 16 wirkt.

Bei der Profildarstellung gemäß der Figur 3b ist zusätzlich die Innentür 6 sowie ein kleiner Teil der Verriegelungs-Zunge 8 zu erkennen; die zur Befestigung des Verschlußelements vorgesehne Oberfläche des Gehäuseteils ist durch eine gestrichelte Linie 20 symbolisch dargestellt.

Eine Schließung der Außentür 16 ist nicht möglich, da deren Innenwand 24 auf den als Distanzelement wirkenden Handgriff 7 stößt, welcher seinerseits an der Außenoberfläche bzw. Außenkontur des Verschlußelements 13 zu liegen kommt und somit einen beabsichtigten Schließvorgang mittels Außentür 4 sperrt; dies bedeutet, daß ein Magnetverschlußteil der Außentür nicht in Berührung mit dem im Gehäuseteil 1 befindlichen Gegenstück treten kann, so daß der magnetische Kreis des Verschlusses nicht geschlossen werden kann.

Anhand Figur 3b ist auch der zum Teil schräg verlaufende bzw. sich verengende Arretierungsspalt 26 erkennbar, der bei Eindrehen der Verriegelungs-Zunge 8 diese in Richtung 27 des symbolisch dargestellten Nutzraumes 2 zwingt, so daß die Innentür gegen das umlaufende Dichtelement 2 gemäß Figur 1 gepreßt wird; die zum Nutzraum gerichtete Nabe des Verriegelungselements 9 ist mit Ziffer 23 bezeichnet.

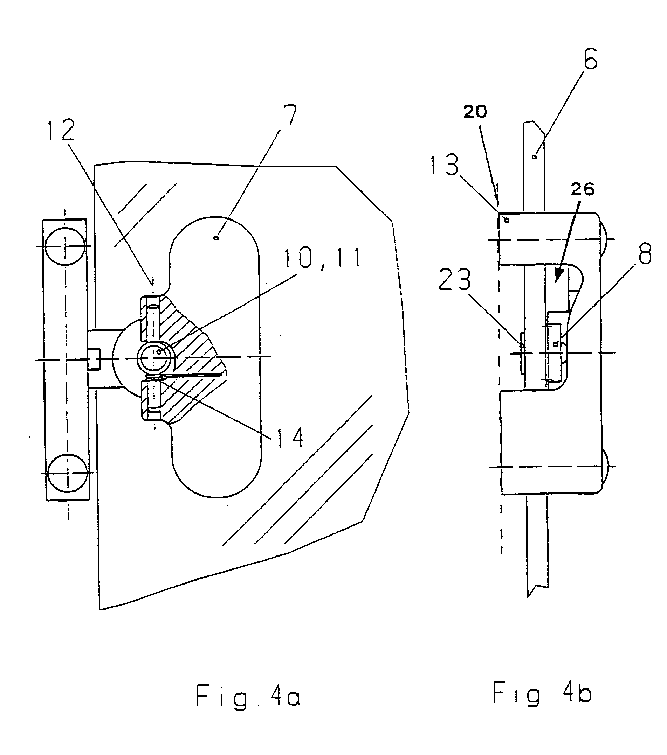

Gemäß Figur 4a ist die Verriegelungs-Zunge 8 entgegen dem Uhrzeiger-Sinn um 90° nach außen gedreht, so daß sie in dem sich verengenden bzw. schräg verlaufenden Arretierungsspalt 26 des Verschlußelements 13 in Richtung Nutzraum gepreßt wird, wobei durch Drehung des Handgriffs 7 um Drehachse 10 in tangentialer Richtung zur Schwenkbewegung der Innentür 6 dieser ebenfalls in Richtung Nutzraum bewegt wird und nach Umklappen mit seiner Außenoberfläche gegen den Federdruck näherungsweise in der gleichen Ebene wie Verschlußelement 13 liegt. Es ist somit gewährleistet, daß in der Verriegelungsposition die Wirkung des Handgriff 7 als Distanzelement verlorengegangen ist und somit Verschlußelement 13 direkt an die Innenwand 24 der Außentür 16 gemäß Figur 2 angrenzt. Somit kann nach der Verriegelung und dem Verlust der Funktion des Distanzelements von Handgriff 7 auch die Außentür 16 sicher verschlossen werden, was bei unverriegelter Innentür 6 keinesfalls möglich wäre.

## Patentansprüche

1. Verfahren zum kontrollierten Verschließen wenigstens einer Innentür und einer Außentür eines Brut- oder Trockenschrankes, **dadurch gekennzeichnet, daß** die mittels seitlichem Scharnier im Öffnungsbereich des Schrankes um Achse (5) schwenkbar gehaltene Innentür (6) auf ihrer der Achse (5) gegenüberliegenden Seite mittels einer mit klappbarem Handgriff (7) versehenen, drehbar gelagerten starren Verschluß-Zunge (8) eines Verriegelungselements (9) durch Drehung der Zunge in einen Arretierungsspalt (26) eines Verschlußelements (13) soweit verriegelt wird, daß der zuvor als Distanzelement zwischen Verschlußelement und Außentür wirkende Handgriff (7) den Auflagebereich des Verschlußelements verläßt und eine zuvor bestehende Blockade zwischen Außentür und Verschlußelement aufgehoben wird.

2. Verfahren nach Anspruch 1, **dadurch gekennzeichnet, daß** beim Einschwenken der Verriegelungs-Zunge (8) in das Verschlußelement (13) diese zusammen mit der Innentür (6) durch einen sich in Verriegelungsrichtung verengenden Arretierungsspalt (26) im Verschlußelement (13) in Richtung Nutzraum gepreßt wird.

3. Brut- oder Trocken-Schrank mit wenigstens einer zwischen Nutzraum und Außentür angeordneten Innentür, wobei beide Türen jeweils durch ein seitliches Scharnier um eine vertikale Schwenk-Achse drehbar gehalten sind, **dadurch gekennzeichnet, daß** zur Verriegelung der Innentür (6) im seitlichen Frontbereich eines Schrank-Gehäuseteils (1) gegenüberliegend der Schwenkachse (5) ein Verschlußelement (13) mit einem sich wenigstens teilsweise verengenden Schlitz als Arretierungsspalt vorgesehen ist, in den eine mit umklappbarem Handgriff (7) versehene, im freien Schwenkbereich der Innentür (6) drehbar gelagerte Verriegelungs-Zunge (8) eines Verriegelungselements (9) eingreifbar angeordnet ist, wobei der Handgriff (7) in der Öffnungsposition der Innentür (6) zwischen Verschlußelement (13) und Innenseite der Außentür als Distanzelement wirkt und ein Schließen der Außentür blockiert, wobei die Verriegelungs-Zunge (8) bei Eingriff in den Schlitz als Arretierungsspalt (26) mittels Drehbewegung bis zum Anschlag innerhalb des Schlitzes so führbar ist, daß sich der Handgriff (7) außerhalb des Auflagebereichs des Verschlußelements (13) befindet und eine Blockade der Außentür (16) bei ihrem Schließvorgang verhindert.

4. Schrank nach Anspruch 3, **dadurch gekennzeichnet, daß** der Handgriff (7) als umklappbares, federbelastetes Distanzelement ausgebildet ist, das diametral gegenüber der Verriegelungs-Zunge (8) angeordnet ist.

5. Schrank nach Anspruch 4, **dadurch gekennzeichnet, daß** der Handgriff (7) im entriegelten Zustand der Verriegelungs-Zunge (8) wenigstens zum Teil als ein zwischen dem Verschlußelement (13) und der Außentür (4) befindliches Distanzelement wirkt.

6. Schrank nach Anspruch 4, **dadurch gekennzeichnet, daß** im verriegelten Zustand der Verriegelungs-Zunge (8) der Handgriff (7) sich in einer Ebene mit dem Verschlußelement (13) befindet.

7. Schrank nach einem der Ansprüche 3 bis 6, **dadurch gekennzeichnet, daß** die Innentür (6) einen transparenten Werkstoff aufweist.

8. Schrank nach Anspruch 7, **dadurch gekennzeichnet, daß** der transparente Werkstoff der Innentür (6) Glas ist.

## Claims

1. A process for controllably locking at least one inner door and one outer door of an incubator or drying oven, **characterized in that**
the inner door (6) which can pivot about axis (5) by means of a lateral hinge within the opening area of the incubator or drying oven is locked on the side opposite its axis (5) by means of a pivoted rigid locking tongue (8) of a latch element (9), the tongue being provided with a foldable handle (7), by the tongue being rotated into a catch opening (26) of a locking element (13),
the inner door (6) being closed to such an extent that handle (7) which previously functioned as a spacing element between the latch element and the outer door leaves the supporting area of the latch element and a previously existing blockade between outer door and latch element is lifted.

2. A process according to claim 1, **characterized in that** during pivoting of locking tongue (8) into locking element (13) the tongue, together with the inner door (6), is forced in the direction of the useful space through a catch opening (26) which narrows in the locking direction.

3. An incubator or drying oven with at least one inner door arranged between the useful space and the outer door, both doors being pivoted about a vertical pivot axis by means of one lateral hinge each, **characterized in that** for locking of the inner door (6) a locking element (13) is provided with an at least partially narrowing slot as a catch opening in the lateral front area of a housing part (1) of an incubator or drying oven opposite the pivot axis (5), which catch opening a locking tongue (8) of a catch element (9) can engage, the locking tongue being provided with a foldable handle (7) and being rotatingly supported within the free pivoting range of the inner door (6), with handle (7) functioning as a spacing element between locking element (13) and the inner side of the outer door and blocking the closing of the outer door when the inner door (6) is in open position, with the locking tongue (8), when engaging the slot as a catch opening (26) via a rotary movement until the stop within the slot, being guidable in such a manner that the handle (7) is positioned outside the support area of the locking element (13) and prevents a blocking of the outer door (16) when it is closing.

4. An incubator or drying oven according to claim 3, **characterized in that** handle (7) is designed as a foldable, spring loaded spacing element which is arranged diametrally opposite locking tongue (8).

5. An incubator or drying oven according to claim 4, **characterized in that** handle (7) in the unlocked state of locking tongue (8) functions at least partially as a spacing element located between locking element (13) and outer door (4).

6. An incubator or drying oven according to claim 4, **characterized in that** handle (7) in the locked state of locking tongue (8) is located in one plane with locking element (13).

7. An incubator or drying oven according to any one of claims 3 to 6, **characterized in that** inner door (6) exhibits a transparent material.

8. An incubator or drying oven according to claim 7, **characterized in that** the transparent material of the inner door (6) is glass.

## Revendications

1. Procédé de fermeture contrôlée d'au moins une porte intérieure et d'une porte extérieure d'une armoire d'incubation ou de séchage, ***caractérisé en ce que*** la porte intérieure (6), tenue pivotante autour d'un axe (5) au moyen d'une charnière latérale dans la zone d'ouverture de l'armoire, est verrouillée du côté opposé à son axe (5) au moyen d'une languette de fermeture (8) rigide d'un élément de verrouillage (9), montée rotative et munie d'une poignée rabattable (7), par rotation de la languette dans une fente d'arrêt (26) d'un élément de fermeture (13), jusqu'à ce que la poignée (7), agissant auparavant comme un élément d'écartement entre l'élément de fermeture et la porte extérieure, quitte la zone d'appui de l'élément de fermeture et qu'un blocage antérieur entre la porte extérieure et l'élément de fermeture soit levé.

2. Procédé selon la revendication 1, ***caractérisé en ce que,*** lors du pivotement de la languette de verrouillage (8) dans l'élément de fermeture (13), celle-ci est pressée avec la porte intérieure (6) en direction de l'espace utile à travers une fente d'arrêt (26) se rétrécissant dans la direction de verrouillage et située dans l'élément de fermeture (13).

3. Armoire d'incubation ou de séchage avec au moins une porte intérieure placée entre un espace utile et une porte extérieure, les deux portes étant tenues rotatives autour d'un axe de pivotement vertical chacune par une charnière latérale, ***caractérisée en ce que***, pour verrouiller la porte intérieure (6), il est prévu dans la zone frontale latérale d'une partie de boîtier (1) de l'armoire, à l'opposé de l'axe de pivotement (5), un élément de fermeture (13) avec une fente se rétrécissant au moins partiellement et servant de fente d'arrêt, dans laquelle est placée de manière insérable une languette de verrouillage (8) d'un élément de verrouillage (9) montée rotative dans la zone de pivotement libre de la porte intérieure (6) et munie d'une poignée rabattable (7), la poignée (7) servant d'élément d'écartement entre l'élément de fermeture (13) et la face intérieure de la porte extérieure dans la position ouverte de la porte intérieure (6) et bloquant une fermeture de la porte extérieure, la languette de verrouillage (8) pouvant, lors de l'insertion dans la fente servant de fente d'arrêt (26), être guidée par un mouvement de rotation jusqu'en butée à l'intérieur de la fente, de sorte que la poignée (7) se trouve en dehors de la zone d'appui de l'élément de fermeture (13) et empêche un blocage de la porte extérieure (16) lors de son opération de fermeture.

4. Armoire selon la revendication 3, ***caractérisée en ce que*** la poignée (7) est conformée en élément d'écartement rabattable monté sur ressort, qui est diamétralement opposé à la languette de verrouillage (8).

5. Armoire selon la revendication 4, ***caractérisée en ce que***, à l'état déverrouillé de la languette de verrouillage (8), la poignée (7) agit au moins en partie comme un élément d'écartement situé entre l'élément de verrouillage (13) et la porte extérieure (4).

6. Armoire selon la revendication 4, ***caractérisée en ce que***, à l'état verrouillé de la languette de verrouillage (8), la poignée (7) se trouve dans un plan avec l'élément de fermeture (13).

7. Armoire selon l'une quelconque des revendications 3 à 6, ***caractérisée en ce que*** la porte intérieure (6) présente un matériau transparent.

8. Armoire selon la revendication 7, ***caractérisée en ce que*** le matériau transparent de la porte intérieure (6) est du verre.
